# EUROPEAN PATENT APPLICATION

(11) **EP 2 857 999 A2**
(43) Date of publication of application: **08.04.2015**
(21) Application number: 14175423.4
(22) Date of filing: 02.07.2014
(51) Int. Cl.: G06F 19/00

(54) **Medical device, reader of the medical device, and method of controlling the medical device**

(30) Priority: 02.10.2013 KR 20130117874
(71) Applicant: Samsung Medison Co., Ltd., Gangwon-do 250-870 (KR)
(72) Inventor: Lee, Sang Mok, Seoul (KR); Hyun, Dong Gyu, Gyeonggi-do (KR); Jo, Jae Moon, Gyeonggi-do (KR); Choi, Seok Won, Gyeonggi-do (KR)
(74) Representative: Schmid, Wolfgang

(57) **Abstract**

Disclosed herein is a medical device including a reader to recognize a command code, and a main body to perform a function matched to the command code recognized by the reader. The medical device may have a small size and be produced at low cost.

## Description

### BACKGROUND

### 1. Field

Embodiments of the present invention relate to a medical device, a reader of the medical device, and a method of controlling the medical device.

### 2. Description of the Related Art

Due to remarkable development of medical technologies, a variety of medical devices are in use today. For example, diagnostic devices such as an X-ray diagnostic device, an X-ray computed tomography (CT) scanner, a magnetic resonance imaging (MRI) device, a nuclear medical diagnostic device, and an ultrasound imaging device are developed and broadly used for rapid and accurate diagnosis of diseases.

In particular, compared to other medical devices, ultrasound imaging devices are small and inexpensive, allow real-time display, and are safe, lacking radiation exposure, and thus are popularized to diagnose heart, abdomen, urological, and gynecologic diseases.

### SUMMARY

Therefore, it is an aspect of the present invention to provide a medical device to perform a function by recognizing a printed or displayed command code, a reader of the medical device, and a method of controlling the medical device.

Additional aspects of the invention will be set forth in part in the description which follows and, in part, will be obvious from the description, or may be learned by practice of the invention.

In accordance with one aspect of the present invention, a medical device includes a reader to recognize a command code, and a main body to perform a function matched to the command code recognized by the reader.

Here, the reader may include a recognition unit to recognize the command code, and a feedback unit to inform that the command code is recognized if the recognition of the command code by the recognition unit has succeeded. The reader may also include an indication unit to indicate the command code to be recognized by the recognition unit.

The medical device may further include a display to display the command code, and the main body may control the display to display the command code. In this case, if the function has sub functions, the main body may control the display to display command codes matched to the sub functions.

The command code may be printed on paper or film.

In accordance with another aspect of the present invention, a reader includes a recognition unit to recognize a command code, and a control unit to control a function matched to the command code recognized by the recognition unit, to be performed.

Here, the command code may be a pattern of text, symbol, barcode, or quick response (QR) code.

In addition, the command code may have a form in which a pattern matched to the function is repeated, the recognition unit may recognize one or more pieces of the repeated pattern, and the control unit may control the function matched to the pattern recognized by the recognition unit, to be performed.

The command code may be displayed on a display, and the recognition unit may recognize the command code displayed on the display. In this case, if the function has sub functions, the reader may control the display to display command codes matched to the sub functions.

The reader may further include a feedback unit to inform that the command code is recognized if the recognition of the command code by the recognition unit has succeeded.

In addition, the reader may further include a command code management unit to match a newly recognized command code to one or more functions to define the command code.

Besides, the reader may further include an indication unit to indicate the command code to be recognized by the recognition unit.

In accordance with another aspect of the present invention, a method of controlling a medical device includes recognizing a command code, and performing a function matched to the command code by analyzing the recognized command code.

In this case, the recognizing may include informing that the command code is recognized if the recognition of the command code has succeeded.

The performing may include, if the function has sub functions, displaying command codes matched to the sub functions.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a block diagram of a medical device including a reader, according to an embodiment of the present invention;
FIGS. 2A and 2B are views for describing command codes according to an embodiment of the present invention;
FIG. 3 is a perspective view of an ultrasound imaging device including a reader, according to an embodiment of the present invention;
FIG. 4 is a detailed block diagram of a reader according to an embodiment of the present invention;
FIG. 5 is a view for describing how to use the reader of FIG. 4;
FIG. 6 is a perspective view of an ultrasound imaging device including a reader, according to another embodiment of the present invention;
FIG. 7 is a detailed block diagram of an ultrasound imaging device according to another embodiment of the present invention;
FIG. 8 is a flowchart of a method of controlling a medical device, according to an embodiment of the present invention; and
FIG. 9 is a flowchart of a method of controlling a medical device, according to another embodiment of the present invention.

### DETAILED DESCRIPTION

Hereinafter, the present invention will be described in detail by explaining embodiments of the invention with reference to the attached drawings. In the following description of the present invention, a detailed description of known functions and configurations incorporated herein will be omitted when it may make the subject matter of the present invention unclear.

The terms used in the specification are defined in consideration of functions used in the present invention, and can be changed according to the intent or conventionally used methods of clients, operators, and users. Accordingly, definitions of the terms should be understood on the basis of the entire description of the present specification.

Furthermore, the embodiments of the present invention described hereinafter are combinations of elements and features of the present invention. The elements or features may be considered selective unless otherwise mentioned. Each element or feature may be practiced without being combined with other elements or features. Further, an embodiment of the present invention may be constructed by combining parts of the elements and/or features.

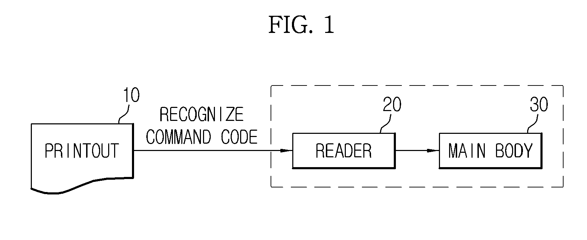

FIG. 1 is a block diagram of a medical device including a reader 20, according to an embodiment of the present invention.

Referring to FIG. 1, the medical device includes the reader 20 and a main body 30. In this case, the medical device should be construed as including all devices used to diagnose or treat people or animals.

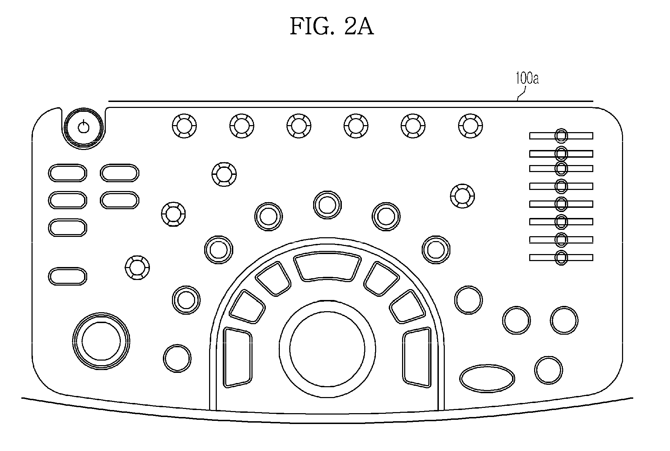

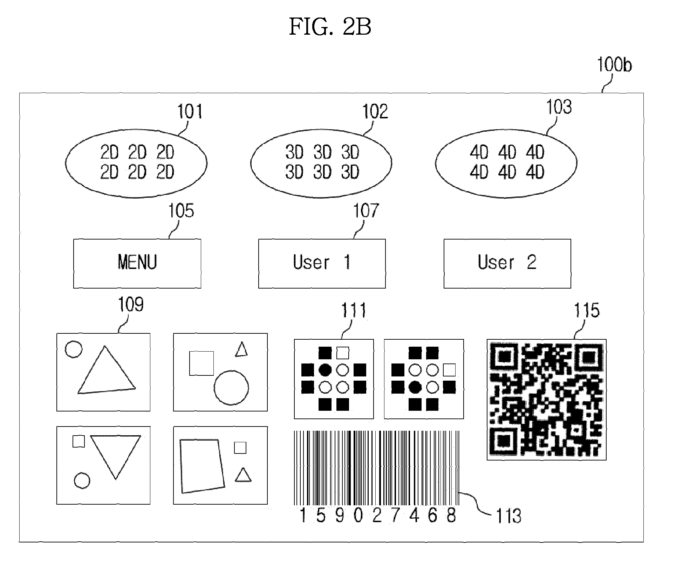

The functions of the medical device may be controlled by the reader 20. The reader 20 controls the function of the medical device by recognizing a printed command code 10. In this case, the command code 10 is a pattern matched to a function of the medical device and will be described in detail below with reference to FIGS. 2A and 2B. In detail, the reader 20 recognizes one of a plurality of printed command codes 10 and controls the medical device to perform a function matched to the recognized command code 10. Therefore, the medical device may be controlled without using a control pad having buttons corresponding to functions, and thus the medical device may have a small size and be produced at low cost. In this case, the reader 20 may have a variety of forms to recognize the command code 10. For example, the reader 20 may have a form of a finger mouse, glasses, or a glove.

The main body 30 performs various functions of the medical device. In this case, the functions performed by the main body 30 may differ in accordance with the type of the medical device. The main body 30 may perform a function in accordance with a control signal of the reader 20 or based on the command code 10 provided from the reader 20.

FIGS. 2A and 2B are views for describing command codes according to an embodiment of the present invention.

Referring to FIG. 1, the command code 10 is a pattern to control the function of the medical device, e.g., the operation or settings of the medical device. In this case, the command code 10 may be a pattern such as text, a symbol, a barcode, or a quick response (QR) code. In detail, a plurality of command codes 10 may be matched to functions of the medical device, and the functions corresponding to the command codes 10 may be different from each other. Optionally, one command code 10 may be matched to a plurality of functions and thus the plurality of functions may be performed when the command code 10 is input.

Referring to FIG. 2A, a printout 100a may include one or more command codes. In this case, the printout 100a may have a form of paper or a film, and the command codes may have been printed on the printout 100a with special paint which is recognizable even when it is contaminated by blood or bodily fluids. For example, the printout 100a may be an output of a printer.

The command codes may be arranged on the printout 100a in the same manner as a conventional fixed reader to increase user adaptability. In this case, the command codes may have a form of text.

Referring to FIG. 2B, command codes 101 to 115 may be arranged on a printout 100b arbitrarily by a user. That is, unlike a hardware reader, the command codes 101 to 115 required to perform functions may be arranged on the printout 100b arbitrarily by the user. Therefore, by arbitrarily arranging the command codes 101 to 115 in consideration of, for example, how frequently functions of a medical device are used, user convenience may be increased.

The command codes 101 to 115 arranged on the printout 100b may have a variety of forms. For example, as illustrated in FIG. 2B, the command codes 101 to 107 may have a form of text, and the command codes 109 and 111 may have a form of figures. The command code 113 may have a form of a barcode formed as a set of vertical bars having different widths. The command code 115 may have a form of a QR code formed as a 2D grid.

Further, each of the command codes 101 to 103 may have a form in which a pattern matched to a function is repeated. In detail, the command code 101 may have a form in which a 2D pattern matched to a function is repeated. If a command code has a form in which a pattern matched to a function of the medical device is repeated as described above, the function may be performed even when only a part of the command code is recognized, and thus a recognition rate of the command code may be increased.

An ultrasound imaging device will now be described in detail as an example of a medical device including a reader. However, the medical device should be construed as not limited to the ultrasound imaging device but including all medical devices equivalent or similar to the ultrasound imaging device.

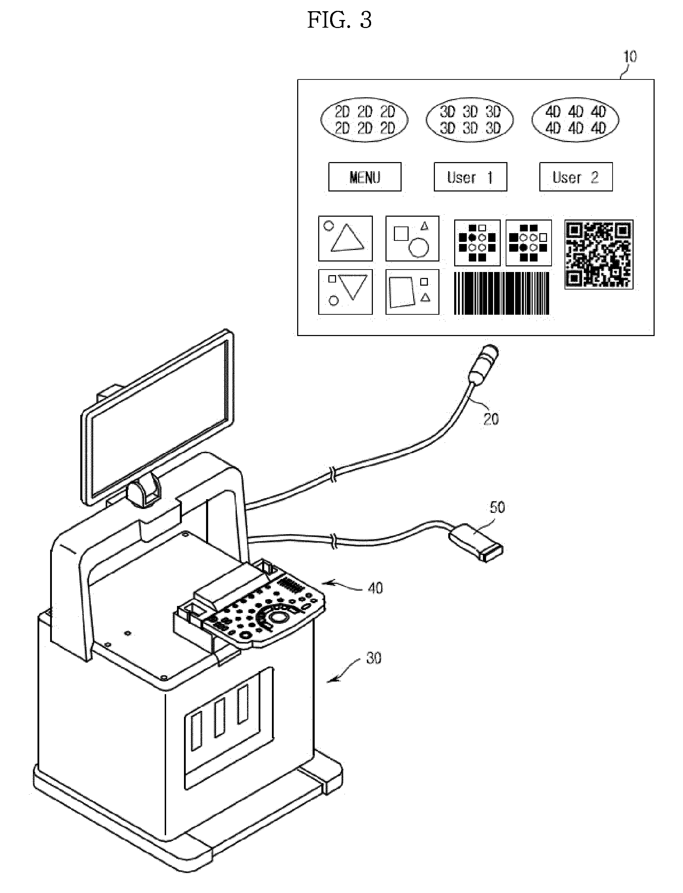

FIG. 3 is a perspective view of an ultrasound imaging device including the reader 20, according to an embodiment of the present invention.

Referring to FIG. 3, the ultrasound imaging device includes the reader 20, the main body 30, a control panel 40, and an ultrasound probe 50.

The reader 20 recognizes the command code 10 and controls the main body 30 to perform a function. The function of the ultrasound imaging device may also be controlled by the control panel 40 of a fixed type. Although the ultrasound imaging device includes both the reader 20 and the control panel 40 in FIG. 3, the ultrasound imaging device may include only the reader 20, or some functions may be performed under the control of the reader 20 while other functions may be performed only under the control of the control panel 40. For example, functions which are not frequently used, e.g., power and preferences, may be controlled by input to the control panel 40, and functions which are frequently used during operation of the ultrasound imaging device, e.g., changing the depth of an ultrasound image, may be controlled by the reader 20.

The ultrasound probe 50 transmits an ultrasound signal to an object to be diagnosed, and receives and provides an ultrasound signal reflected from the object, to the main body 30. The main body 30 generates an ultrasound image using the ultrasound signal received from the ultrasound probe 50. In this case, the main body 30 may perform a function under the control of the reader 20 or the control panel 40, and may control operation of the ultrasound probe 50 if necessary.

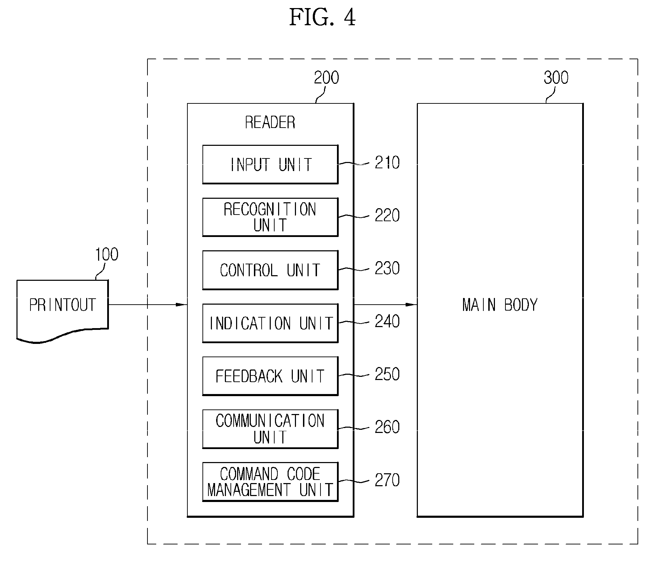

FIG. 4 is a detailed block diagram of a reader 200 according to an embodiment of the present invention.

Referring to FIG. 4, the reader 200 includes an input unit 210, a recognition unit 220, a control unit 230, an indication unit 240, a feedback unit 250, a communication unit 260, and a command code management unit 270. In this case, one or more command codes may have been printed on a printout 100. For example, the command codes 101 to 115 may be arranged as illustrated in FIG. 2B.

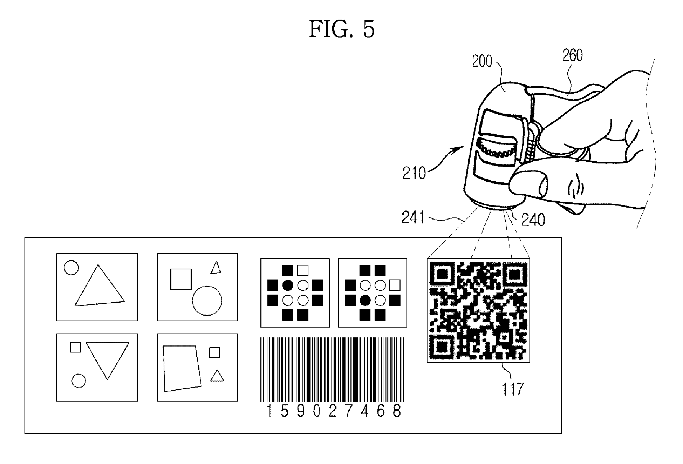

The input unit 210 receives a command code recognition command of a user. The input unit 210 may receive the command code recognition command in a variety of manners. For example, the input unit 210 may receive the command code recognition command based on a certain motion of the user as well as manipulation of a certain button or a wheel (see 210 of FIG. 5) by the user.

The recognition unit 220 recognizes a command code. In detail, the recognition unit 220 recognizes one of the command codes which have been printed on the printout 100. The recognition unit 220 may include, for example, a camera or optical sensor to recognize the command code. For example, the recognition unit 220 may include an optical character reader (OCR). Here, the OCR is a device to read characters using light, and more particularly, to a device to irradiate light to text, symbols, or marks printed or handwritten on paper and convert reflected light therefrom into an electrical signal. Alternatively, the recognition unit 220 may be formed as a barcode reader or a QR code reader to recognize the command code.

If the recognized command code has a form in which a pattern matched to a function is repeated, the recognition unit 220 may extract the repeated pattern from the command code. In this case, like the command code, the extracted pattern is also matched to the function. Therefore, if the recognized command code has a form in which a pattern matched to a function is repeated, even when only a part of the command code is recognized, the recognition unit 220 may extract the repeated pattern from the partially recognized command code and thus may achieve the effect of recognizing the whole command code. Therefore, by configuring a command code to have a form in which a pattern is repeated, a recognition rate of the command code may be increased. For example, in the case of the command code 101 of FIG. 2, the recognition unit 220 may extract a repeated pattern, 2D from a recognized part of the command code 101, and may recognize the extracted 2D as the command code 101.

In addition, the recognition unit 220 may perform a variety of operations to accurately recognize a command code. For example, the recognition unit 220 may adjust the focus of a camera to recognize a command code.

The control unit 230 controls a function corresponding to the command code recognized by the recognition unit 220, to be performed. In detail, the control unit 230 analyzes a function matched to the command code recognized by the recognition unit 220, and generates a control signal corresponding to the analyzed function to be performed. In this case, the control signal may be transmitted via the communication unit 260 to the main body 300. For example, if the recognized command code corresponds to a function of generating a 3D ultrasound image, the control unit 230 may transmit a control signal to perform a 3D ultrasound imaging function, via the communication unit 260 to the main body 300.

The indication unit 240 indicates a part to be recognized if an input control signal is generated. In detail, the indication unit 240 indicates, on the printout 100, a region to be recognized by the recognition unit 220 if the input control signal is generated. For example, the indication unit 240 may have a form of a laser pointer to indicate the region to be recognized by the recognition unit 220 if the input control signal is generated.

The feedback unit 250 provides feedback if the command code is recognized by the recognition unit 220. In detail, if the recognition unit 220 recognizes the command code, the feedback unit 250 informs, using vibration, sound, or light, a user of an ultrasound imaging device that the recognition of the command code has succeeded. For example, the feedback unit 250 may be formed as a motor to generate vibration, a speaker to output sound, or a lamp to generate light. In addition, the feedback unit 250 informs that the recognition of the command code has failed if the command code is not recognized within a certain time.

The communication unit 260 transmits control signals to and receives control signals from the main body 300. In detail, the communication unit 260 transmits the control signal generated by the control unit 230 to the main body 300. In this case, the communication unit 260 may transmit control signals to and receive control signals from the main body 300 in accordance with a variety of wired/wireless communication protocols. For example, a short-range communication protocol such as wireless local access network (WLAN), Bluetooth, or ZigBee may be used.

The command code management unit 270 matches command codes to functions. In detail, the command code management unit 270 matches a new command code input by the user to one or more functions providable by the ultrasound imaging device. In this case, the command code management unit 270 may provide an interface to match the command codes to the functions.

The main body 300 performs a function in accordance with the above-described control signal of the reader 200. In addition, in order to inform that the control signal has been successfully received, the main body 300 may provide feedback when the control signal is received from the reader 200 and the function is performed. For example, the main body 300 may provide voice to indicate the function corresponding to the recognized command code.

FIG. 5 is a view for describing how to use the reader 200 of FIG. 4.

Referring to FIGS. 4 and 5, the user of the ultrasound imaging device may control the function of the ultrasound imaging device based on the reader 200 having a form of a finger mouse. In this case, the finger mouse is connected to and is communicable with the main body 300 via a cable as the communication unit 260, and includes the input unit 210 to receive a command code recognition command from the user.

The indication unit 240 may indicate, using a laser pointer 241 to project a square-shaped laser, a QR code 117 corresponding to a region to be recognized by the recognition unit 220.

If the user inputs the command code recognition command via the input unit 210, the recognition unit 220 of the reader 200 recognizes the QR code 117. In this case, if the recognition of the QR code 117 has succeeded, the feedback unit 250 may provide, for example, vibration or sound to the user.

Although the reader 200 has a form of a finger mouse in the above description, the command code recognition command may be received from the user in a variety of manners in accordance with the form of the reader 200. For example, if the reader 200 has a form of glasses, the command code recognition command of the user may be a blink of an eye(s). Otherwise, if the reader 200 has a form of a glove, the command code recognition command may be a motion of a finger of the user.

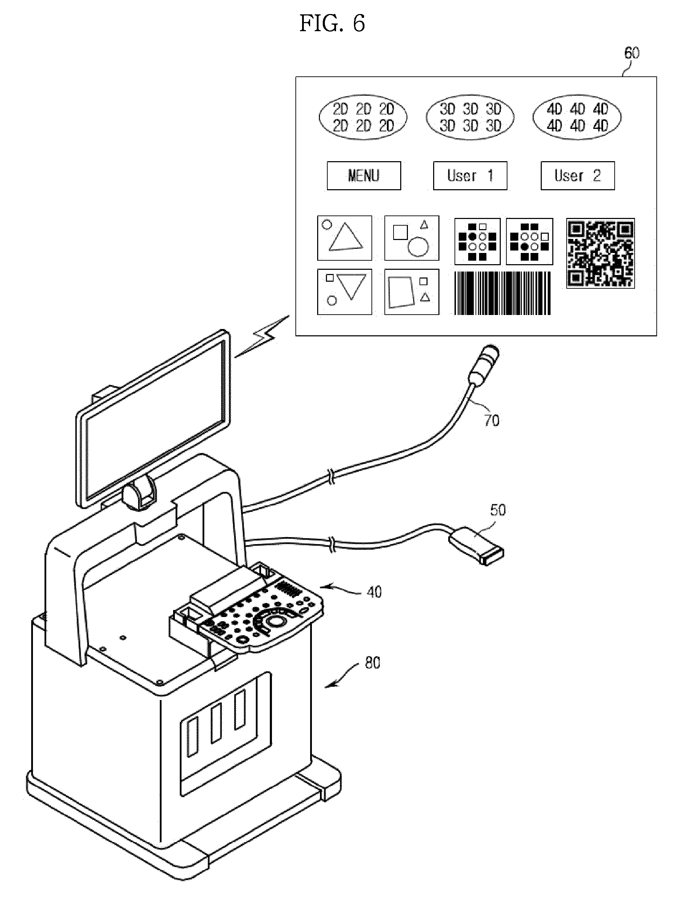

FIG. 6 is a perspective view of an ultrasound imaging device including a reader 70, according to another embodiment of the present invention.

Referring to FIG. 6, the ultrasound imaging device includes the control panel 40, the ultrasound probe 50, a display 60, the reader 70, and a main body 80.

Compared to the FIG. 3, the ultrasound imaging device may further include the display 60 to display command codes. As such, the command codes may be output more flexibly.

In this case, the display 60 includes all devices to display command codes, which may be recognized by the reader 70, at a certain location.

In detail, the display 60 displays certain command codes under the control of the main body 80 or the reader 70. For example, the display 60 may be a display device such as a liquid crystal display (LCD), a light-emitting diode (LED), an organic light-emitting diode (OLED), an active matrix organic light-emitting diode (AMOLED), a flexible display, or a 3D display.

In addition, the display 60 may be any of various devices to receive and display command codes from the reader 70 or the main body 80. For example, the display 60 may be a device to receive command codes from the reader 70 or the main body 80 via a communication device such as a laptop computer, a touch pad, a tablet computer, or a smartphone, and display the received command codes.

Alternatively, the display 60 may be a display device to display command codes at a certain location, for example, a projector or a laser.

The reader 70 or the main body 80 may control the display 60 to display command codes. In detail, the reader 70 or the main body 80 may determine command codes to be displayed on the display 60, and may control the display 60 to display the determined command codes.

If a recognized command code has sub command codes, the reader 70 or the main body 80 may control the display 60 to display the sub command codes. For example, if the recognized command code corresponds to a "settings" function of the ultrasound imaging device, the reader 70 or the main body 80 may control the display 60 to display command codes matched to sub functions "depth", "brightness", etc. of the "settings" function.

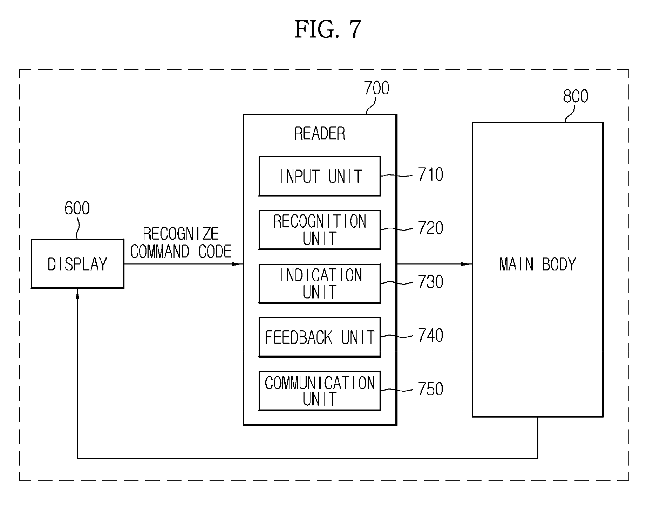

FIG. 7 is a detailed block diagram of an ultrasound imaging device according to another embodiment of the present invention;

Referring to FIG. 7, a reader 700 recognizes a command code displayed on a display 600, and transmits the recognized command code to a main body 800. In detail, if a command code recognition command is input via an input unit 710, a recognition unit 720 recognizes and transmits the command code via a communication unit 750 to the main body 800. In this case, a feedback unit 740 may generate feedback upon successful recognition of the command code. An indication unit 730 may indicate, on the display 600, a part to be recognized by the recognition unit 720.

The main body 800 performs a function matched to the command code recognized by the reader 700. In detail, the main body 800 receives the command code from the reader 700, analyzes a function corresponding to the received command code, and performs the analyzed function.

The main body 800 may control the display 600 to display the command code. In this case, the command code displayed on the display 600 may differ in accordance with every situation, and may be changed by the control of a user or in accordance with performance of the function.

Although the feedback unit 740 provides the feedback upon successful recognition of the command code in the above description, alternatively, the main body 800 may provide the feedback to the user. In detail, if the recognized command code is received, the main body 800 may provide, to the user, feedback to inform that the command code is recognized. For example, the main body 800 may provide the feedback using sound.

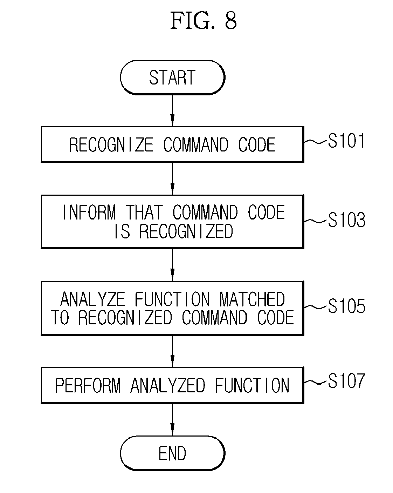

FIG. 8 is a flowchart of a method of controlling a medical device, according to an embodiment of the present invention; and

Referring to FIG. 8, the medical device recognizes a command code in S101. In detail, the medical device may recognize the command code using a reader including, for example, a camera or an optical sensor, and more particularly, a character reader, a barcode reader, or a QR code reader. In addition, if the command code is formed as a repeated pattern, the medical device may extract the repeated pattern from the recognized command code, and may recognize the extracted pattern as the command code. In this case, if the recognition of the command code has failed, the medical device may attempt the recognition of the command code again after adjusting the focus of the camera or the optical sensor.

If the command code is recognized in S101, the medical device generates feedback upon successful recognition of the command code in S103. In this case, the feedback may have a form of sound, light, or vibration and may inform a user that the command code is recognized.

The medical device analyzes a function matched to the recognized command code in S105, and performs the analyzed function in S107.

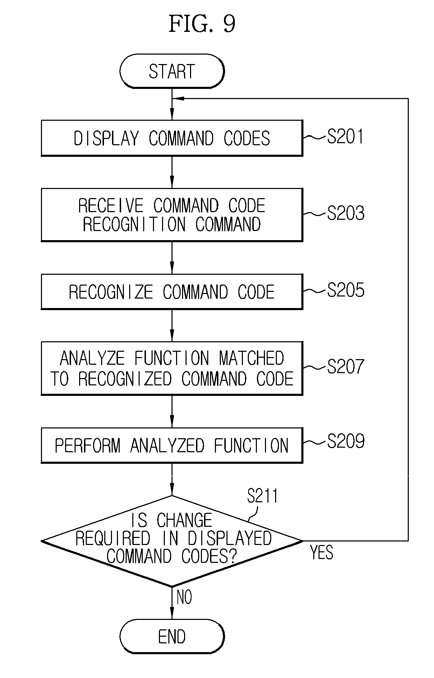

FIG. 9 is a flowchart of a method of controlling a medical device, according to another embodiment of the present invention.

Referring to FIG. 9, the medical device displays command codes in S201. In detail, the medical device determines command codes to be displayed on a display, and controls the display to display the determined command codes. In this case, the display may display a plurality of command codes, and the command codes to be displayed may be selected or arranged by a user.

If a command code recognition command is input by the user in S203, the medical device recognizes a command code in S205. In this case, the medical device may indicate the command code to be recognized. In addition, the medical device may provide, to the user, feedback to inform that recognition of the command code has succeeded. If the recognition of the command code within a certain time after the command code recognition command is input, the medical device may also provide feedback to inform that recognition of the command code has failed.

The medical device analyzes a function matched to the recognized command code in S207, and performs the analyzed function in S209.

The medical device determines whether to change the displayed command codes in S211. In detail, the medical device determines whether to display new command codes in accordance with the performance of the function in S207.

Upon determining to change the displayed command codes in S211, the medical device returns to S201 to change the displayed command codes.

As is apparent from the above description, since functions of a medical device may be performed based on command codes, the functions of the medical device may be performed even when the medical device does not include a fixed control panel. Therefore, the medical device may have a small size and be produced at low cost.

Furthermore, since the command codes used to perform the functions of the medical device may be arranged arbitrarily by a user of the medical device, user convenience may be improved. Besides, the command codes and the functions of the medical device may be matched arbitrarily by the user, individualized functions may be easily performed.

In addition, as the command codes may be printed on inexpensive and replaceable paper or a film, the medical device may be used sanitarily by replacing the paper or film.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A medical device comprising:
a reader to recognize a command code; and
a main body to perform a function matched to the command code recognized by the reader.

2. The medical device according to claim 1, wherein the reader comprises:
a recognition unit to recognize the command code; and
a feedback unit to inform that the command code is recognized if the recognition of the command code by the recognition unit has succeeded.

3. The medical device according to claim 1, wherein the reader comprises:
a recognition unit to recognize the command code; and
an indication unit to indicate the command code to be recognized by the recognition unit.

4. The medical device according to claim 1, further comprising a display to display the command code,
wherein the main body controls the display to display the command code.

5. The medical device according to claim 4, wherein, if the function has sub functions, the main body controls the display to display command codes matched to the sub functions.

6. The medical device according to claim 1, wherein the command code is printed on paper or film.

7. A reader comprising:
a recognition unit to recognize a command code; and
a control unit to control a function matched to the command code recognized by the recognition unit, to be performed.

8. The reader according to claim 7, wherein the command code is a pattern of text, symbol, barcode, or quick response (QR) code.

9. The reader according to claim 7, wherein the command code has a form in which a pattern matched to the function is repeated,
wherein the recognition unit recognizes one or more pieces of the repeated pattern, and
wherein the control unit controls the function matched to the pattern recognized by the recognition unit, to be performed.

10. The reader according to claim 7, wherein the command code is displayed on a display, and
wherein the recognition unit recognizes the command code displayed on the display.

11. The reader according to claim 7, further comprising a feedback unit to inform that the command code is recognized if the recognition of the command code by the recognition unit has succeeded.

12. The reader according to claim 7, further comprising an indication unit to indicate the command code to be recognized by the recognition unit.

13. A method of controlling a medical device, the method comprising:
recognizing a command code; and
performing a function matched to the command code by analyzing the recognized command code.

14. The method according to claim 13, wherein the recognizing comprises informing that the command code is recognized if the recognition of the command code has succeeded.

15. The method according to claim 13, wherein the performing comprises, if the function has sub functions, displaying command codes matched to the sub functions.
